# EUROPÄISCHE PATENTSCHRIFT

(11) **EP 2 008 615 B1**
(45) Veröffentlichungstag und Bekanntmachung des Hinweises auf die Patenterteilung: **21.11.2012**
(21) Anmeldenummer: 08011631.2
(22) Anmeldetag: 26.06.2008
(51) Int. Cl.: A61F 2/06

(54) **Sinusflicken zum Ersatz von defekten Sinus an der Aortenwurzel**
Sinus patch for replacing defective sinuses on the aortic root
Rustine de sinus destinée au remplacement d'un sinus défectueux de l'anneau aortique

(30) Priorität: 27.06.2007 DE 102007031146
(43) Veröffentlichungstag der Anmeldung: 31.12.2008
(73) Patentinhaber: Aesculap AG, 78532 Tuttlingen (DE)
(72) Erfinder: Goldmann, Helmut, 34119 Kassel (DE); Merckle, Christof, 68199 Mannheim (DE); Lippoth, Lisa, 78588 Denkingen (DE)
(74) Vertreter: Patentanwälte Ruff, Wilhelm, Beier, Dauster & Partner

(56) Entgegenhaltungen:
- EP-A1- 1 935 375
- WO-A1-2005/099624
- GB-A- 2 312 485
- US-A- 5 139 515
- US-A1- 2003 139 805

## Beschreibung

Die Erfindung betrifft Sinusflicken zum Ersatz von defekten Sinus an der Aortenwurzel bestehend aus einem flächigen, flexiblen, gewölbten Wandungsmaterialstück.

Bei Herzoperationen im Bereich der Aortenklappe und der Aortenwurzel gibt es verschiedene Methoden, je nach dem wie groß der Defekt in diesem Bereich ist. Ist die Aortenklappe als solche noch intakt aber die Aortenwurzel schadhaft, dann können Aortenprothesen eingesetzt werden, die die Sinus bzw. Bulbi im Bereich der Aortenwurzel bereits aufweisen. Es ist auch möglich ganze Aortenbogenprothesen mit den Sinus zu implantieren.

Aus der WO 01/52776 A1 ist eine Aortenwurzelprothese bekannt, die an ihrem herznahen Ende drei Sinus aufweist. Diese Sinus werden aus flachem Gefäßprothesenmaterial in der geeigneten Form ausgeschnitten.

Die Sinusflicken werden dann an ihrem Außenrand mit einem Faden gesäumt und gerafft, wodurch eine dreidimensionale Wölbung erhalten wird. Danach werden sie mit dem unteren Rand der Aortensinuswurzelprothese durch Annähen verbunden.

Aus der US 5,139,515 ist eine Aortenprothese mit einem aortenwurzelseitigen, bulbusförmigen Abschnitt und einem sich daran anschließenden aortenbogenseitigen, zylindrischen Abschnitt bekannt.

Der Erfindung liegt die Aufgabe zugrunde, die Anwendungsmöglichkeiten von Sinusflicken zu verbessern. Gemäß der Erfindung ist es vorgesehen, dass die Sinusflicken aus einem flächigen, flexiblen, gewölbten Wandungsmaterialstück gebildet sind, das, abgesehen von einer etwaigen Plissierung, eine dreidimensionale Wölbung aufweist, die durch das Wandungsmaterial selbst bedingt ist. Die Wölbung ist eine materialimmanente Eigenschaft der Wandung. Dies bedeutet, dass die Sinusflicken aus einem von vorneherein eine dreidimensionale Wölbung besitzenden Wandungsmaterial gebildet sind. Dadurch entfällt einerseits der Arbeitsschritt des Einsäumens und Raffens. Auf der anderen Seite wird dadurch die Möglichkeit gewonnen, die Form und Größe der Sinusflicken unmittelbar vor der Implantation zu verändern bzw. zu korrigieren, ohne dass die Wölbung dadurch beeinträchtigt wird. So können die Sinusflicken beispielsweise am Rand beschnitten werden, was bei den durch Einsäumen und Raffen gebildeten Sinusflicken nicht möglich ist. Besonders geeignet sind die Sinusflicken auch zum unmittelbaren Ersatz von defekten Sinus bzw. Bulbi, d.h. auch für solche Operationen, bei denen die natürliche Aortensinuswurzel erhalten bleibt und lediglich die Bulbi ausgetauscht werden.

Bei einer Ausführungsform der Erfindung sind die Sinusflicken Ausschnitte aus einer dreidimensional gewölbten Wandung, insbesondere Gefäßprothesenwandung. Die Wölbung ist vorzugsweise vorgeformt. Dies ist dadurch möglich, dass ein ursprünglich flaches oder nur zweidimensional vorgeformtes Wandungsmaterial verformt wird, um eine dreidimensionale Wölbung zu erhalten. Es ist auch möglich, die dreidimensionale Wölbung bereits bei der Herstellung des Wandungsmaterials vorzuformen.

So sind bei einer Ausführungsform der Erfindung die Sinusflicken Ausschnitte aus einer Wandung an der Außenseite einer Aortenbogenprothese mit einer materialimmanenten, insbesondere fixierten Krümmung. Solche Ausschnitte haben aufgrund der Form der Aortenbogenprothesen an ihrer Außenseite bereits eine dreidimensionale Wölbung. Es ist bei einer anderen Ausführungsform auch möglich, die Sinusflicken als Ausschnitte aus einer toroidförmigen Wandung, insbesondere Gefäßprothesenwandung vorzusehen. Auch hier ist die dreidimensionale Wölbung materialimmanent.

Die Sinusflicken können aus einem textilen Wandungsmaterial bestehen. Dieses kann beispielsweise gewebt oder gewirkt sein. In der Regel wird ein Wandungsmaterial verwendet, wie es auch für Gefäßprothesen üblich ist. Es ist auch möglich, die Sinusflicken aus einem non-woven Material auszubilden. Besonders bevorzugt sind hier Sprühvliese, insbesondere solche aus unvernetztem bzw. linearem Polyurethan. Wandungen aus non-woven Material können in einfacher Weise schon mit der gewünschten Wölbung hergestellt werden.

Die Sinusflicken können in an sich bekannter Weise eine Plissierung aufweisen. Die Plissierung kann in Querrichtung verlaufen, wie dies bei Gefäßprothesen normalerweise der Fall ist. Die Plissierung kann aber auch, was bevorzugt ist, in Längsrichtung der Sinusflicken verlaufen, die normalerweise im wesentlichen der Längsrichtung der Aortenwurzel entspricht. Solche Plissierungen können dazu dienen, die Formstabilität und/oder Dehnbarkeit der Sinusflicken zu erhöhen.

Die Sinusflicken besitzen bevorzugt eine größere Länge als Breite, wie dies auch bei den natürlichen Sinus der Fall ist. Die Form ist in der Regel etwa wappen- bis schildförmig. Wie bereits gesagt, kann die genaue Form jeweils durch entsprechendes Zuschneiden bzw. Beschneiden erhalten werden.

Die toroidförmige Wandung, insbesondere der toroidförmige Gefäßprothesenabschnitt, kann aus textilem oder non-woven-Material bestehen. Bevorzugt ist sie als textiles Gewebe ausgebildet. Dabei kann ein toroidförmiger Gewebeschlauch über den Schlauchumfang gesehen, im Bereich der Wölbung eine konstante Kettfadenzahl aufweisen. Dies bedeutet, dass die Aufwölbung durch sich vergrößernde Abstände der Kettfäden gebildet ist. Es ist aber auch möglich, beim toroidförmigen Schlauch die Wölbung dadurch zu erzeugen, dass im Bereich der Wölbung zusätzliche Kettfäden zugeführt werden, d.h. der toroidförmige Schlauchabschnitt eine zunehmende und wieder abnehmende Kettfadenzahl in Bezug auf den Umfang aufweist.

Bei einer anderen Ausführungsform kann zunächst von einem zylindrischen oder konischen Schlauch ausgegangen werden, der dann durch Schrumpfung und/oder Dehnung des Wandungsmaterials, insbesondere eines textilen Wandungsmaterials in die gewölbte Form überführt wird, wobei die gewölbte Form durch eine Fixierung stabilisiert wird, beispielsweise durch thermische Fixierung.

Wie bereits erwähnt, kann die Wölbung auch dadurch erzeugt werden, dass die Sinusflicken durch Materialaufbau auf einen dreidimensional geformten Kern ausgebildet werden. Eine solche Ausbildung eignet sich besonders bei als Sprühvlies ausgebildeten Sinusflicken.

Das Wandungsmaterial der Sinusflicken ist vorzugsweise porös. Wie bei Gefäßprothesen dient eine solche Porosität dazu, das Einwachsen von Bindegewebe zu ermöglichen. Weiterhin können die Sinusflicken auch beschichtet oder imprägniert sein, insbesondere mit einem resorbierbaren Material, um eine Abdichtung des Wandungsmaterials zu erhalten.

Gemäß der Erfindung können die Sinusflicken in verschiedenen Größen vorliegen, beispielsweise in Abhängigkeit von der Größe der zu ersetzenden Sinus. Die Sinusflicken können auch bei im wesentlichen gleicher Länge eine verschiedene Breite besitzen, was dann von Vorteil ist, wenn ein Patient eine asymmetrische Aortenklappe besitzt, bei der die einzelnen Sinus verschieden groß sind. Auch hier ist es möglich, vor der Implantation noch gewünschte Korrekturen vorzunehmen.

Weitere Merkmale der Erfindung ergeben sich aus der nachfolgenden Beschreibung von bevorzugten Ausführungsformen in Verbindung mit den Unteransprüchen und der Zeichnung. Hierbei können die einzelnen Merkmale jeweils für sich alleine oder in Kombination miteinander verwirklicht sein.

### In der Zeichnung zeigen

- Figur 1:: Ausführungsformen der Erfindung in Form von Ausschnitten aus Aortenbogenprothesen;
- Figur 2:: ein Schnitt entlang der Linien II-II nach Figur 1;
- Figur 3:: weitere Ausführungsformen in Form von Ausschnitten aus einer toroidförmigen Gefäßprothese;
- Figur 4:: eine weitere Ausführungsform nach der Erfindung und
- Figur 5:: weitere Ausführungsformen nach der Erfindung.

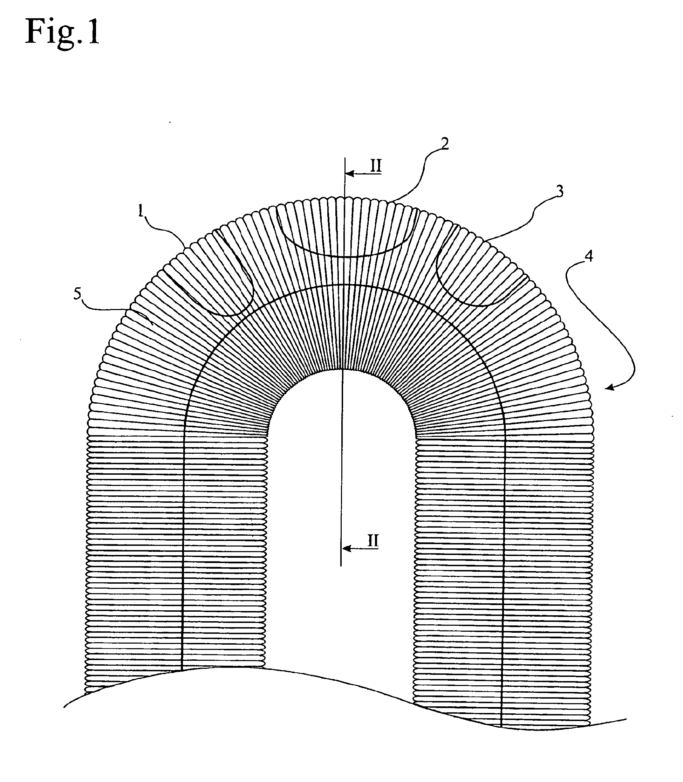

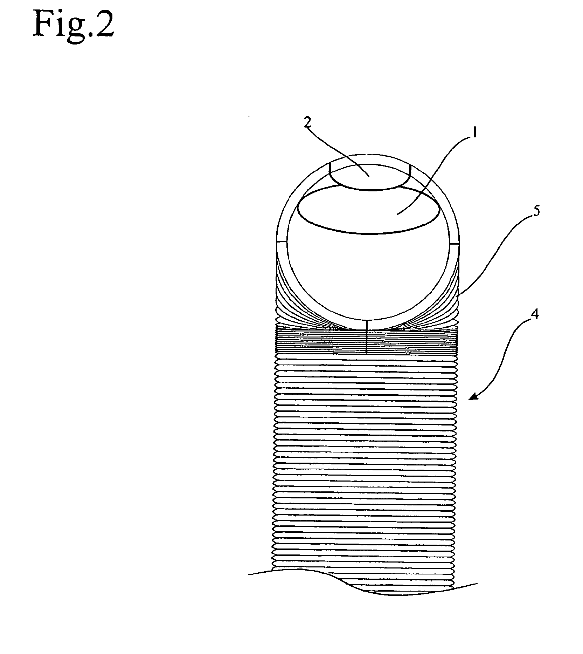

Bei den Ausführungsformen nach den Figuren 1 und 2 sind Sinusflicken 1, 2, 3 in Form von Ausschnitten aus der Außenwandung im Außenradius einer Aortenbogenprothese 4 ausgebildet. Die Aortenbogenprothese 4 ist eine textile Aortenbogenprothese aus gewebtem oder gewirktem Material. Sie weist eine Plissierung 5 auf. Die Aortenbogenprothese 4 ist aus einer schlauchförmigen zylindrischen Prothese mit Plissierung hergestellt. Die Bogenform ist dadurch stabilisiert, dass die Prothese in gebogener Form thermisch fixiert wurde. Die Aortenbogenprothese 4 kann aber auch durch ein Sprühvlies gebildet sein, das auf einem entsprechend gebogenen Kern, der ebenfalls eine Plissierung aufweisen kann, abgeformt ist. Je nach dem in welcher Weise die Sinusflicken aus der Aortenbogenprothese ausgeschnitten werden, können Sinusflicken mit einer Längsplissierung (1 und 3) oder mit einer Querplissierung (2) erhalten werden. Es können schmale oder breite Sinusflicken ausgeschnitten werden. Je nach dem inwieweit noch die Seitenwandungen der Aortenbogenprothese mit einbezogen werden, können auch Sinusflicken mit asymmetrischer Wölbung erhalten werden.

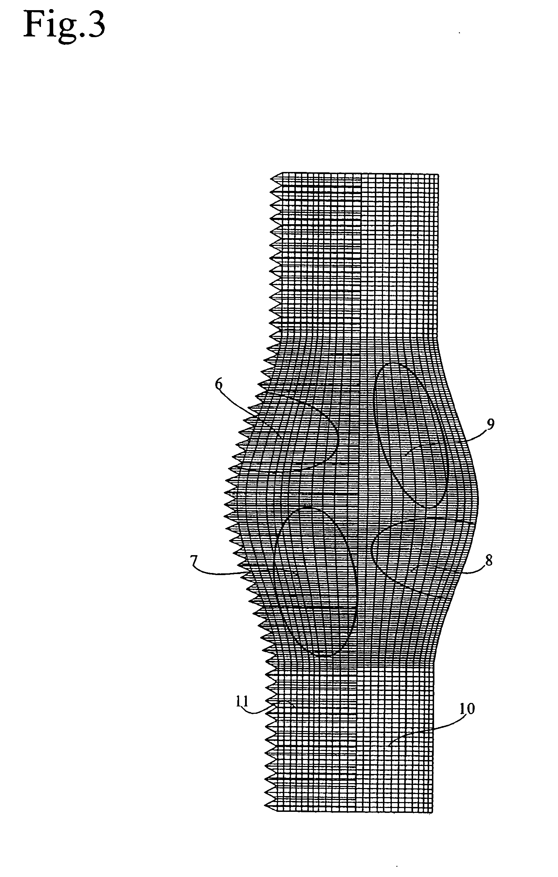

Bei der Ausführungsform nach Figur 3 sind Sinusflicken 6, 7, 8 und 9 als Ausschnitte aus einer toroidförmigen Gefäßprothese 10 ausgebildet. Die Gefäßprothese ist vorzugsweise eine gewebte Gefäßprothese mit einer konstanten Anzahl von Kettfäden über die Wölbung hinweg. Die Gefäßprothese kann, wie in der einen Längshälfte gezeigt, eine Plissierung 11 aufweisen. Wie gezeigt, können die Sinusflicken je nach Art des Ausschnitts wiederum eine Querplissierung oder eine Längsplissierung aufweisen. Die Gefäßprothese kann, wie in der zweiten Längshälfte dargestellt, auch unplissiert sein. Je nach Ausschnitt sind auch hier unterschiedliche Formen und Wölbungen möglich. Die toroidförmige Gefäßprothese kann auch in einfacher Weise in Form eines Sprühvlieses, beispielsweise aus Polyurethan, ausgebildet sein. Dementsprechend sind dann auch die Sinusflicken ausgebildet. Weiterhin ist es möglich, die Wölbung, insbesondere die toroidförmige Wölbung durch Dehnen und/oder Schrumpfen einer Gefäßprothese auf einen toroidförmigen Kern auszubilden.

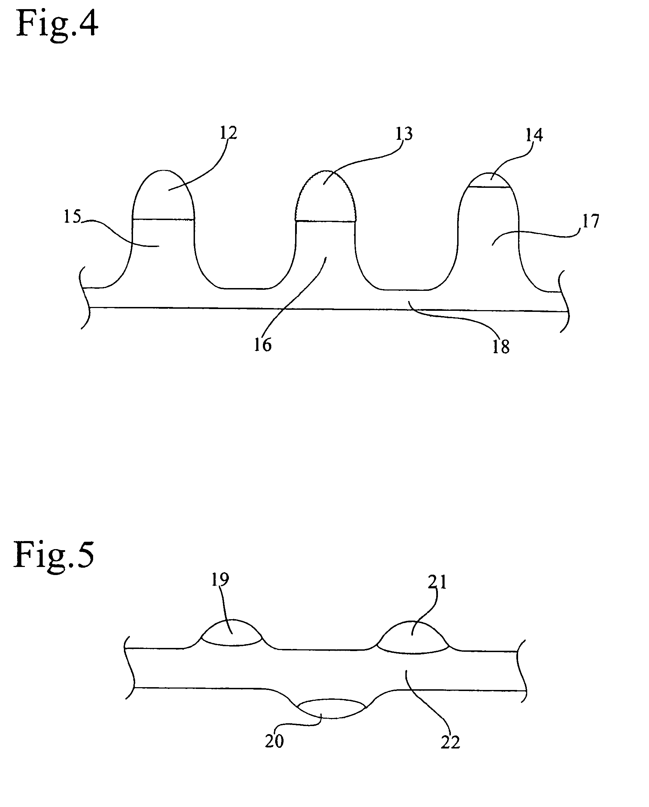

Bei der Ausführungsform nach Figur 4 sind Sinusflicken 12, 13, 14 vorgesehen, die als Polyurethansprühvlies ausgebildet sind. Die Wölbung ist dadurch erhalten, dass eine Höcker 15, 16, 17 aufweisende Grundplatte 18 als Unterlage für die Ausbildung eines Sprühvlieses diente. Die Höcker 15, 16 und 17 können unterschiedliche Form haben. Dementsprechend sind dann auch die Sinusflicken 12, 13 und 14 unterschiedlich ausgebildet. Auch hier können Plissierungen vorgesehen sein, wie dies beim Höcker 16 und dem Sinusflicken 13 angedeutet ist.

Bei der Ausführungsform nach Figur 5 sind Sinusflicken 19, 20, 21 vorgesehen, die wiederum als Sprühvlies ausgebildet sind. Die Form wird dadurch erhalten, dass eine Nockenwalze 22 bei langsamer Umdrehung besprüht wurde. Auch hier sind unterschiedliche Formen und beliebige Anzahl von Nocken möglich.

Die Wandungen der Sinusflicken 19, 20, 21 können auch dadurch ausgebildet werden, dass die Nockenwalze in eine Polyurethanlösung getaucht wird und man die dabei erhaltene Polyurethanschicht auf der Nockenwalze unter Drehung der Walze trocknen lässt.

Bei sämtlichen Ausführungsformen ist das Wandungsmaterial der Sinusflicken von sich aus bereits gewölbt, ohne dass hierzu besondere Hilfsmittel notwendig sind. Dadurch ist es möglich, die Sinusflicken in beliebiger Größe und Form herzustellen bzw. auszuschneiden. Die Sinusflicken können mit einer Aortenwurzelprothese kombiniert werden oder unmittelbar zum Ersatz von defekten Sinus in die natürliche Aortenwurzel eingesetzt werden.

## Patentansprüche

1. Sinusflicken (1, 2, 3; 6, 7, 8, 9; 12, 13, 14; 19, 20, 21) zum Ersatz von defekten Sinus der Aortenwurzel bestehend aus einem flächigen, flexiblen, gewölbten Wandungsmaterialstück, das abgesehen von einer etwaigen Plissierung (5; 11), eine dreidimensionale Wölbung aufweist, die durch das Wandungsmaterial selbst bedingt ist.

2. Sinusflicken nach Anspruch 1, **dadurch gekennzeichnet, dass** sie von einem Ausschnitt aus einer dreidimensional gewölbten Wandung, insbesondere Gefäßprothesenwandung (4; 10) gebildet werden.

3. Sinusflicken nach Anspruch 1 oder 2, **dadurch gekennzeichnet, dass** sie von einem Ausschnitt aus einer Wandung an der Außenseite einer Aortenbogenprothese (4) mit einer materialimmanenten, insbesondere fixierten Krümmung gebildet werden.

4. Sinusflicken nach Anspruch 1 oder 2, **dadurch gekennzeichnet, dass** sie von einem Ausschnitt aus einem toroidförmigen Gefäßprothesenabschnitt (10) gebildet werden.

5. Sinusflicken nach einem der vorhergehenden Ansprüche, **dadurch gekennzeichnet, dass** sie aus einem textilen Wandungsmaterial bestehen.

6. Sinusflicken nach einem der Ansprüche 1 bis 4, **dadurch gekennzeichnet, dass** sie aus einem non-woven Material gebildet sind.

7. Sinusflicken nach Anspruch 6, **dadurch gekennzeichnet, dass** sie von einem Sprühvlies gebildet werden.

8. Sinusflicken nach einem der vorhergehenden Ansprüche, **dadurch gekennzeichnet, dass** sie eine Plissierung (5; 11) aufweisen.

9. Sinusflicken nach einem der vorhergehenden Ansprüche, **dadurch gekennzeichnet, dass** sie eine größere Länge als Breite besitzen.

10. Sinusflicken nach Anspruch 8 oder 9, **dadurch gekennzeichnet, dass** Falten der Plissierung (5) in Längsrichtung des Sinusflicken (1, 3) verlaufen.

11. Sinusflicken nach einem der Ansprüche 4 bis 10, **dadurch gekennzeichnet, dass** der toroidförmige Gefäßprothesenabschnitt (10) aus einem textilen Gewebe besteht.

12. Sinusflicken nach Anspruch 11, **dadurch gekennzeichnet, dass** das textile Gewebe im toroiden Gefäßabschnitt eine konstante Kettfadenzahl aufweist.

13. Sinusflicken nach Anspruch 11, **dadurch gekennzeichnet, dass** der toroidförmige Gefäßabschnitt eine zunehmende und abnehmende Kettfadenzahl aufweist.

14. Sinusflicken nach einem der vorhergehenden Ansprüche, **dadurch gekennzeichnet, dass** die dreidimensionale Wölbung durch Schrumpfung und/oder Dehnung des Wandungsmaterials, insbesondere eines textilen Wandungsmaterials, gebildet ist.

15. Sinusflicken nach einem der Ansprüche 1 bis 9, **dadurch gekennzeichnet, dass** sie durch Materialaufbau auf einen dreidimensional geformten Kern gebildet sind.

16. Sinusflicken nach einem der vorhergehenden Ansprüche, **dadurch gekennzeichnet, dass** das Wandungsmaterial porös ist.

## Claims

1. Sinus patches (1, 2, 3; 6, 7, 8, 9; 12, 13, 14; 19, 20, 21) for replacing defective sinuses of the aortic root, consisting of a flat, flexible, domed piece of wall material which, in addition to possibly having pleats (5; 11), has a three-dimensional dome shape, which is determined by the wall material itself.

2. Sinus patches according to Claim 1, **characterized in that** they are formed from a section cut from a three-dimensionally domed wall, especially a vascular prosthesis wall (4; 10).

3. Sinus patches according to Claim 1 or 2, **characterized in that** they are formed from a section cut from a wall at the outer side of an aortic arch prosthesis (4) having a curvature that is inherent to the material, and particularly a fixed curvature.

4. Sinus patches according to Claim 1 or 2, **characterized in that** they are formed by a section cut from a toroidal-shaped vascular prosthesis segment (10).

5. Sinus patches according to one of the previous claims, **characterized in that** they are made from a textile wall material.

6. Sinus patches according to one of Claims 1 to 4, **characterized in that** they are formed from a nonwoven material.

7. Sinus patches according to Claim 6, **characterized in that** they are formed from a spray-bonded web.

8. Sinus patches according to one of the previous claims, **characterized in that** they have pleats (5; 11).

9. Sinus patches according to one of the previous claims, **characterized in that** they are longer than they are wide.

10. Sinus patches according to Claim 8 or 9, **characterized in that** folds of the pleats (5) run in the longitudinal direction of the sinus patches (1, 3).

11. Sinus patches according to one of Claims 4 to 10, **characterized in that** the toroidal-shaped vascular prosthesis segment (10) is made from a woven textile substrate.

12. Sinus patches according to Claim 11, **characterized in that** the woven textile in the toroidal-shaped vascular segment has a constant number of warp threads.

13. Sinus patches according to Claim 11, **characterized in that** the toroidal-shaped vascular segment has an increasing and decreasing number of warp threads.

14. Sinus patches according to one of the previous claims, **characterized in that** the three-dimensional dome is produced by shrinking and/or stretching the wall material, especially a textile wall material.

15. Sinus patches according to one of Claims 1 to 9, **characterized in that** they are formed by depositing material onto a three-dimensionally shaped core.

16. Sinus patches according to one of the previous claims, **characterized in that** the wall material is porous.

## Revendications

1. Rustine de sinus (1, 2, 3; 6, 7, 8, 9; 12, 13, 14; 19, 20, 21) destinée au remplacement d'un sinus défectueux de l'anneau aortique, se composant d'une pièce de matière de paroi incurvée, flexible, en nappe qui, indépendamment d'un éventuel plissage (5; 11), présente une courbure tridimensionnelle, qui est déterminée par la matière de paroi elle-même.

2. Rustine de sinus selon la revendication 1, **caractérisée en ce qu'**elle est formée par un fragment tiré d'une paroi incurvée tridimensionnelle, en particulier d'une paroi de prothèse vasculaire (4, 10).

3. Rustine de sinus selon la revendication 1 ou 2, **caractérisée en ce qu'**elle est formée par un fragment tiré d'une paroi sur le côté extérieur d'une prothèse de l'arche aortique (4) avec une courbure immanente de la matière, en particulier une courbure fixe.

4. Rustine de sinus selon la revendication 1 ou 2, **caractérisée en ce qu'**elle est formée par un fragment tiré d'une partie toroïdale de prothèse vasculaire (10).

5. Rustine de sinus selon l'une quelconque des revendications précédentes, **caractérisée en ce qu'**elle se compose d'une matière de paroi textile.

6. Rustine de sinus selon l'une quelconque des revendications 1 à 4, **caractérisée en ce qu'**elle est formée en une matière non tissée.

7. Rustine de sinus selon la revendication 6, **caractérisée en ce qu'**elle est formée d'un non-tissé du type spray-bonded.

8. Rustine de sinus selon l'une quelconque des revendications précédentes, **caractérisée en ce qu'**elle présente un plissage (5; 11).

9. Rustine de sinus selon l'une quelconque des revendications précédentes, **caractérisée en ce qu'**elle présente une longueur plus grande que la largeur.

10. Rustine de sinus selon la revendication 8 ou 9, **caractérisée en ce que** des plis du plissage (5) s'étendent dans la direction longitudinale de la rustine de sinus (1, 3).

11. Rustine de sinus selon l'une quelconque des revendications 4 à 10, **caractérisée en ce que** la partie toroïdale de prothèse vasculaire (10) se compose d'un tissu textile.

12. Rustine de sinus selon la revendication 11, **caractérisée en ce que** le tissu textile présente un nombre de fils de chaîne constant dans la partie vasculaire toroïdale.

13. Rustine de sinus selon la revendication 11, **caractérisée en ce que** la partie vasculaire toroïdale présente un nombre croissant et décroissant de fils de chaîne.

14. Rustine de sinus selon l'une quelconque des revendications précédentes, **caractérisée en ce que** la courbure tridimensionnelle est formée par le retrait et/ou la dilatation de la matière de paroi, en particulier d'une matière de paroi textile.

15. Rustine de sinus selon l'une quelconque des revendications 1 à 9, **caractérisée en ce qu'**elle est formée par dépôt de matière sur un noyau moulé tridimensionnel.

16. Rustine de sinus selon l'une quelconque des revendications précédentes, **caractérisée en ce que** la matière de la paroi est poreuse.
